# EUROPEAN PATENT APPLICATION

(11) **EP 1 378 567 A1**
(43) Date of publication of application: **07.01.2004**
(21) Application number: 02712969.1
(22) Date of filing: 10.04.2002
(51) Int. Cl.: C12N 9/16, C12N 15/55, C12N 1/21, A01H 5/00

(54) **BACTERIAL ADPGLUCOSE PYROPHOSPHATASE, METHOD FOR OBTAINING THE LATTER AND ITS USE IN THE PRODUCTION OF ASSAY DEVICES AND IN THE OBTENTION OF TRANSGENIC PLANTS AND BACTERIA**

(30) Priority: 10.04.2001 ES 200100844
(71) Applicant: UNIVERSIDAD PUBLICA DE NAVARRA, 31006 Pamplona (ES)
(72) Inventor: POZUETA ROMERO, Javier, 31006 Pamplona (ES); BAROJA FERNANDEZ, Edurne, 31006 Pamplona (ES); MUNOZ PEREZ, Francisco, José, 31006 Pamplona (ES); MORENO BRUNA, Beatriz, 31006 Pamplona (ES); ZANDUETA CRIADO, Aitor, 31006 Pamplona (ES); RODRIGUEZ LOPEZ, Milagros, 31006 Pamplona (ES); BASTARRICA BERASATEGUI, Ainara, 31006 Pamplona (ES); MORAN ZORZANO, Maria, Teresa, 31006 Pamplona (ES)
(74) Representative: Elzaburu Marquez, Alberto
(86) International application number: PCT/ES2002/000174
(87) International publication number: WO 2002/081672

(57) **Abstract**

Bacterial ADPglucose pyrophosphatase, method of production, use in the manufacture of testing devices and in the production of transgenic plants and bacteria.

AGPPase is an enzyme that catalyzes the hydrolysis of ADPG (adenosine diphosphate glucose). The enzyme obtained from microbial extracts is used in testing devices for determining levels of ADPG, based on the G1P (glucose-1-phosphate) released by the reaction catalyzed by AGPPase. Partial amino acid sequences of the enzyme, the sequence of the gene that codes for it and the derived protein are also described. Finally, the production of transgenic plants and bacteria that overexpress the gene of AGPPase is described. The bacteria do not accumulate glycogen, whereas the plants obtained possess a high content of soluble sugars, low starch content and high resistance to high concentrations of salts and to high temperatures.

## Description

### FIELD OF TECHNOLOGY TO WHICH THE INVENTION RELATES

The invention relates to the field of the production, purification and characterization of the enzyme ADPglucose pyrophosphatase (AGPPase), its use for making devices (kits) for determining ADPG in both plant and microbial extracts, and the production of transgenic plants and bacteria in which there is overexpression of the gene that codes for AGPPase, aspP, giving rise to plants and bacteria with reduced content of starch or glycogen, respectively, high content of soluble sugars and high resistance to salinity and to high temperatures.

### STATE OF THE PRIOR ART

Both glycogen and starch are storage forms of carbohydrates in bacteria and plants, respectively. In plants, starch accumulates in large quantities in organs such as seeds (wheat, barley, maize, peas, etc.) and tubers (potato and yam (sweet potato), among others), and is a basic constituent of the human diet. On the other hand, starch is often used in the paper, cosmetics, pharmaceutical and food industries, and is also used as a basic component for the manufacture of biodegradable plastics and environmentally friendly paints. As these polysaccharides are made up of covalently-bonded glucose molecules, investigation of the processes involved in their synthesis is an urgent matter in various fields of industrial production.

ADPglucose (ADPG) is the universal precursor in the biosynthesis of the starch in plants and of bacterial glycogen. Considerations concerning the factors that govern the intracellular levels of ADPG have mainly focused on the regulation of the synthesizing enzyme, ADPG pyrophosphorylase (Preiss (1988), "Biosynthesis of starch and its regulation". The Biochemistry of Plants. Vol. 14, Academic Press, New York, pp. 182-249; Pozueta-Romero, J., Perata, P., Akazawa, T. (1999) "Sucrose-starch conversion in heterotrophic tissues". Crit. Rev. Plant Sci., 18, 489-525; Preiss, J. (1984) Bacterial glycogen synthesis and its regulation. Ann. Rev. Microbiol. 38, 419-458; Preiss, J., Romeo, T. (1994) Molecular biology and regulatory aspects of glycogen biosynthesis in bacteria, Prog. Nucleic Acid Res. Mol. Biol. 47, 301-327). However, there has been little investigation of possible mechanisms of degradation of ADPG that help to modulate the levels of starch and glycogen accumulated by the cell (Feingold, D.S., Avigad, G. (1980) "Sugar transformation in plants". The Biochemistry of Plants. Vol. 3, Stumpf, P.K. and Conn, E.E. Eds. Academic Press, New York, pp. 101-170) and there are few indications that both bacteria and plants possess an enzymatic machinery capable of hydrolyzing ADPG (Melo, A., Glaser, L. (1966) "Nucleotide diphosphate hexose pyrophosphatases". Biochem. Biophys. Res. Commun. 22, 524-531; Rodríguez-López, M., Baroja-Fernández, E., Zandueta-Criado, A., Pozueta-Romero, J. (2000) "Adenosine diphosphate glucose pyrophosphatase: a plastidial phosphodiesterase that prevents starch biosynthesis". Proc. Natl. Acad. Sci., 97, 8705-8710; Baroja-Fernández, E., Zandueta-Criado, A., Rodriguez-López, M., Akazawa, T., Pozueta-Romero, J. (2000) "Distinct isoforms of ADPglucose pyrophosphatase and ADPglucose pyrophosphorylase occur in the suspension-cultured cells of sycamore (Acer pseudoplatanus L.). FEBS Lett. 480, 277-282; Rodríguez-López, M., Baroja-Fernández, E., Zandueta-Criado, A., Moreno-Bruna, B., Muñoz, F.J., Akazawa, T., Pozueta-Romero, J. (2001) "Two isoforms of a nucleotide-sugar pyrophosphatase/phosphodiesterase from barley leaves (Hordeum vulgare L.) are distinct oligomers of HvGLP1, a germin-like protein". FEBS Lett. 490, 44-48; Moreno-Bruna, B., Baroja-Fernández, E., Muñoz, F.J., Bastarrica-Berasategui, A., Zandueta-Criado, A., Rodríguez-López, M., Lasa, I., Akazawa, T., Pozueta-Romero, J. (2001) "Adenosine diphosphate sugar pyrophosphatase prevents glycogen biosynthesis in Escherichia coli" Proc. Natl. Acad. Sci. 98, 8128-8132).

The various applications of starch produced in a plant are based mainly on the balance of amylose and amylopectin, which determines the structure of the starch grain, as well as its viscosity in aqueous suspensions. This ratio of amylose and amylopectin depends on the concentration of ADPG in the plant cell (Clarke, B.R., Denyer, K., Jenner, C.F., Smith, A.M. (1999) The relationship between the rate of starch synthesis, the adenosine 5'-diphosphoglucose concentration and the amylose content of starch in developing pea embryos. Planta 209, 324-329).

As well as acting as a reserve substance for the plant, starch accumulates in the plant cell in circumstances in which the plant is not submitted to conditions of water stress. In conditions where the plant is subjected to high temperatures or high concentrations of salts in the environment, the plant ceases to accumulate starch, producing large quantities of soluble sugars which accumulate in the vacuole (Keeling, P.L., Bacon, P.J., Holt, D.C. (1993) "Elevated temperature reduces starch deposition in wheat endosperm by reducing the activity of soluble starch synthase" Planta 191, 342-348; Geigenberger, P., Geiger, M., Stitt, M. (1998) "High-temperature perturbation of starch synthesis is attributable to inhibition of ADP-glucose pyrophosphorylase by decreased levels of glycerate-3-phosphate in growing potato tubers" Plant Physiol. 117, 1307-1316).

The invention describes the purification and applications of an enzyme product of microbial origin that we call AGPPase, which catalyzes the hydrolysis of ADPG. In the present invention we succeeded in partially sequencing the AGPPase of Escherichia coli. After comparing the sequenced fragments with the sequences available in the databases, it is concluded that AGPPase is a protein belonging to the group of "Nudix hydrolases" encoded by the sequence of the genome of E. coli designated orf209 whose product had not until now been identified in the cell and to which the possible function of regulating the content of ADPribose therein has been attributed (Bessman, M.J., Frick, D.N., O'Handley, S.F. (1996) "The MutT proteins or "Nudix" hydrolases, a family of versatile, widely distributed "housecleaning" enzymes" J. Biol. Chem. 271, 25059-25062; Dunn, C.A., O'Handley, S.F., Frick, D.N., Bessman, M.J. (1999) "Studies on the ADP-ribose pyrophosphatase subfamily of the Nudix hydrolases and tentative identification of trgB, a gene associated with tellurite resistance" J. Biol. Chem. 274, 32318-32334). The accession number of aspP (the gene that codes for AGPPase and was previously designated with the name orf209) available in the EMBL database is: AJ298136.

Chromatographic techniques constitute a powerful tool for determining the content of ADPG in plant and microbial extracts (Sweet love, L.J., Burrell, M.M., ap Rees, T. (1996) "Starch metabolism in tubers of transgenic potato (Solanum tuberosum) with increased ADPglucose pyrophosphorylase" Biochem. J. 320, 493-498; Shannon, J.C., Pien, F-M., Liu, K-C. (1996) "Nucleotides and nucleotide sugars in developing maize endosperms" Plant Physiol. 110, 835-843; Geigengerger, P., Reimboltz, R., Geiger, M., Merlo, L., Canale, V., Stiff, M. (1997) "Regulation of sucrose and starch metabolism in potato tubers in response to short-term water deficit" Plant 201, 520-518; Geigenberger, P., Geiger, M., Stitt, M. (1998) High-temperature perturbation of starch synthesis is attributable to inhibition of ADPglucose pyrophosphorylase by decreased levels of glycerate-3-phosphate in growing potato tubers" Plant Physiol. 117, 1307-1316). Although of very general application, they require a considerable investment in equipment. Unfortunately, scant use is made of possible alternative methods that permit the detection and quantification of ADPG in a simple and efficient manner. Obviously the possibility of analyzing the levels of ADPG in a sample in a simple and inexpensive manner represents an advantageous alternative to the chromatographic techniques.

The invention relates, firstly, to the production of AGPPase in substantially pure form, starting from E. coli, its characterization and identification of the gene that encodes it. Another object of the invention is the procedure followed for making devices or kits for determination of ADPG based on the use of the enzyme product with AGPPase activity. Having identified the gene that codes for AGPPase, aspP, details are given of the design of DNA constructions derived therefrom, intended for the production of transgenic plants and bacteria with high AGPPase activity whose content and quality of starch or glycogen, respectively, are modified relative to the control plants. The transformed plants, monocotyledonous or dicotyledonous, display resistance to high salinity and temperatures. The transformed bacteria can be employed in hand feeding, particularly in aquaculture.

### DETAILED DESCRIPTION OF THE INVENTION

### Production and purification of AGPPase from E. coli

AGPPase is present in various strains of E. coli (BL21, ATCC12793 and K12 among others), Salmonella typhimurium and Haemophilus influenzae. The general method of production and purification of AGPPase described in the invention is illustrated using E. coli BL21 and includes the following steps, in which small changes can be made that do not substantially alter the general scheme of the method of extraction and purification:
1. Centrifugation at 10,000 g, of 30 liters of a bacterial suspension grown to a steady state in M9 minimum medium (96 mM Na2HPO4, 44 mM KH2PO4, 15 mM NaCl, 35 mM NH4Cl, 0.1 mM CaC12, 2 mM MgSO4 with 5 mM of glucose.
2. The bacteria are resuspended in 200 ml of 50 mM Tris-HCl pH 7.5 and, after sonication, are ultracentrifuged at 100,000 g for 30 minutes.
3. The supernatant is heated at 58°C for 10 minutes and is centrifuged at 30,000 g for 30 minutes.
4. The proteins of the supernatant are precipitated in 30-50% of ammonium sulfate and are resuspended in 2.7 ml of 50 mM Tris-HCl pH 7.5.
5. The sample is applied to a gel filtration column of type Superdex 200 (Pharmacia LKB), pre-equilibrated with 50 mM Tris-HCl pH 7.5/150 mM NaCl.
6. The fractions showing AGPPase activity are concentrated, dialyzed and applied to a Mono-Q column (HR 5x5, Pharmacia) equilibrated with 50 mM Tris-HCl pH 7.5. The enzyme is eluted with a gradient of 45 ml of 0-1 M KCl in 30 mM Tris-HCl pH 8.0.
7. The active fractions are submitted to a step of isoelectric focusing using a Rotofor Cell (Bio-Rad).

### Identification of the product with enzymatic activity AGPPase

The enzyme product obtained by the procedures described above, or other equivalent procedures, is identified by means of the following functional standards:
- It is an "adenosine diphosphate-sugar pyrophosphatase" (EC 3.6.1.21) which catalyzes the hydrolysis of ADPG to equimolar amounts of G1P and AMP.
- In addition to ADPG, it recognizes ADPmannose and ADPribose, but does not recognize other nucleotide sugars such as UDPglucose, GDPmannose, UDPglucuronic, etc. Nor does it recognize nucleotides such as 5'-phosphosulfate, ATP, ADP, UTP, GTP nor the artificial substrate bis-paranitrophenyl-phosphate used for the characterization of phosphodiesterases.
- It does not hydrolyze molecules with phosphomonoester bonds such as G1P, G6P, AMP, 3-phosphoglycerate, and the like. Nor does it hydrolyze cyclic AMP or long-chain nucleic acids such as DNA or RNA.
- It is inhibited by phosphorylated molecules such as AMP, ADP, ATP, 3-phosphoglycerate, inorganic pyrophosphate, and others with similar characteristics.
- It is strongly inhibited by molybdate.
- Its activity is affected by the action of β-mercaptoethanol, EDTA, reduced cysteine, ascorbate, and other reducing and chelating agents.
- It displays maximum activity in a pH range between 7.5 and 8.5.

### Amplification of the gene (aspP) of E. coli that codes for AGPPase

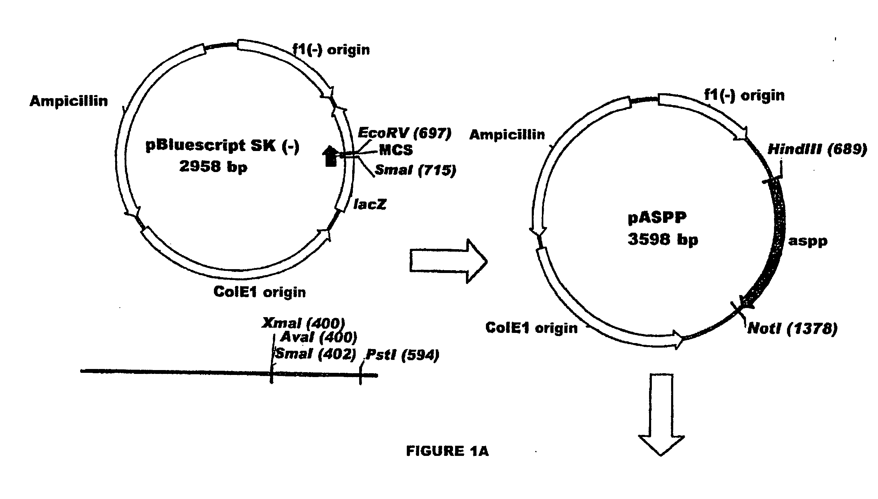

Once the amino acid sequence of AGPPase was known it was compared with others present in databases. This made it possible to identify the gene that codes for AGPPase, to which the name aspP has been assigned. Knowledge of the nucleotide sequence of aspP made it possible to create two specific primers. Using these primers, a DNA of 640 base pairs was amplified by conventional PCR methods from the genomic DNA of E. coli BL21, which was introduced between the EcoRV/SmaI restriction sites of the plasmid pSK Bluescript (Stratagene) giving rise to the construction pASPP (Fig. 1A) which was amplified in the XL1 Blue host bacterium.

### Production of AGPPase from E. coli that overexpresses aspP

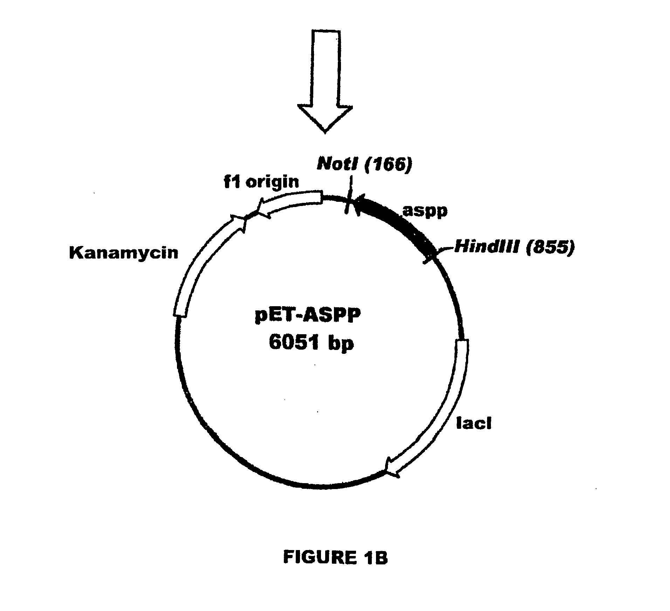

pASPP was digested with the restriction enzymes HindIII and NotI. The fragment released (which contains aspP) was cloned in the pET-28a(+) expression vector (Novagen), giving rise to the plasmid pET-ASPP (Fig. 1B) which was introduced by electroporation in E. coli BL21 (DE3) . The strain of E. coli was deposited in the Spanish Collection of Standard Cultures on 10.5.2000, located in the Research Building of the University of Valencia, Campus de Burjassot, Burjassot 46100 (Valencia, Spain) with the deposition No. CECT5357. The overexpression of aspP took place by addition of 1 mM isopropyl-β-D-thiogalactopyranoside (IPTG) in 100 ml of cellular culture. After six hours of induced culture the bacteria were collected and were resuspended in 4 ml of binding buffer (Novagen, His-bind purification kits), and were sonicated and centrifuged at 40,000 g for twenty minutes. The supernatant, containing AGPPase with a glue of histidines, was passed through an affinity column of the "His-bind" protein purification kit from Novagen. Following the instructions for the kit, AGPPase was eluted with 6 ml of the recommended elution buffer.

### Production of transgenic plants that overexpress aspP

### a. Plants that overexpress aspP constitutively and accumulate AGPPase in the cytosol

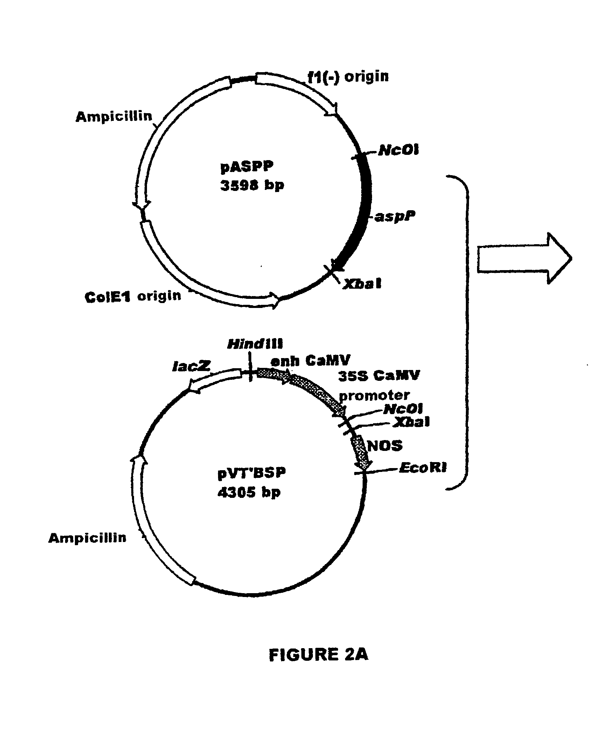

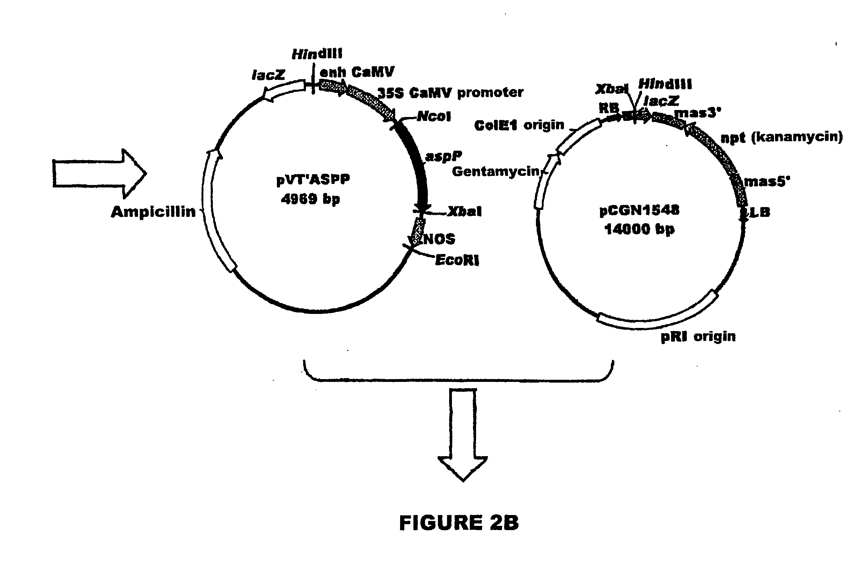

pASPP was digested with the enzymes NcoI and XbaI. The fragment released (which contains aspP) was cloned in the plasmid pVT'BSP at the NcoI/XbaI sites (Fig. 2A). In this way a plasmid called pVT'ASPP is obtained which possesses the constitutive promoter 35S, aspP and the terminator Nos (Fig. 2B).

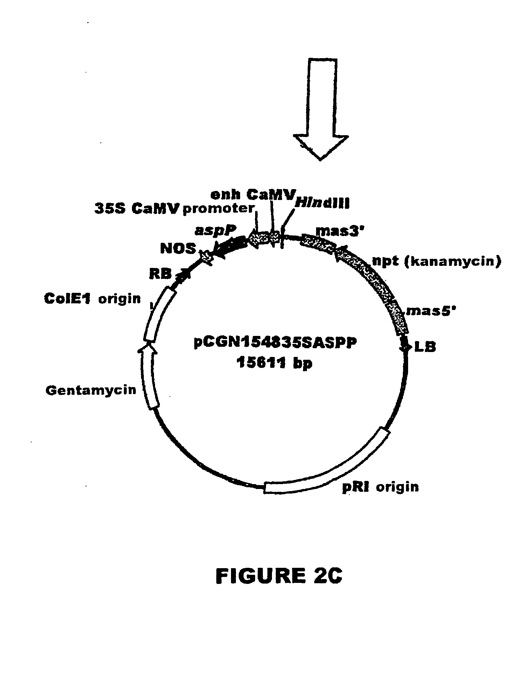

In order to be able to transfer this construction to the plants' genome via Agrobacterium tumefaciens, it must first be cloned in a binary plasmid. For this, pVT'ASPP was digested successively with the enzymes EcoRI, T4 DNA polymerase and HindIII and was cloned within the binary plasmid pCGN1548 (McBride, K.E., Summerfelt, K.R. (1990) "Improved binary vectors for Agrobacterium-mediated plant transformation". Plant Mol. Biol. 14, 269-276) which had previously been digested successively with the enzymes XbaI, T4 DNA polymerase and HindIII. The plasmid thus obtained was designated pCGN154835SASPP (Fig. 2C). After being amplified in E. coli (XL1 Blue), pCGN154835SASPP was introduced into Agrobacterium tumefaciens (CECT 5901) which was used for transforming species such as tomato, tobacco, potato, etc. (Horsch, R.B., Fry, J.E., Hoffmann, N.L., Eichholtz, D., Rogers, S.G., Fraley, R.T. (1985) "A simple and general method for transferring genes into plants" Science 277, 1229-1231. The strain of Agrobacterium tumefaciens was deposited in the Spanish Collection of Standard Cultures on 02.16.2001, located in the Research Building of the University of Valencia, Campus de Burjassot, Burjassot 46100 (Valencia, Spain) with the deposition No. CECT 5901.

### b. Plants that overexpress aspP exclusively in tubers and accumulate AGPPase in the cytosol

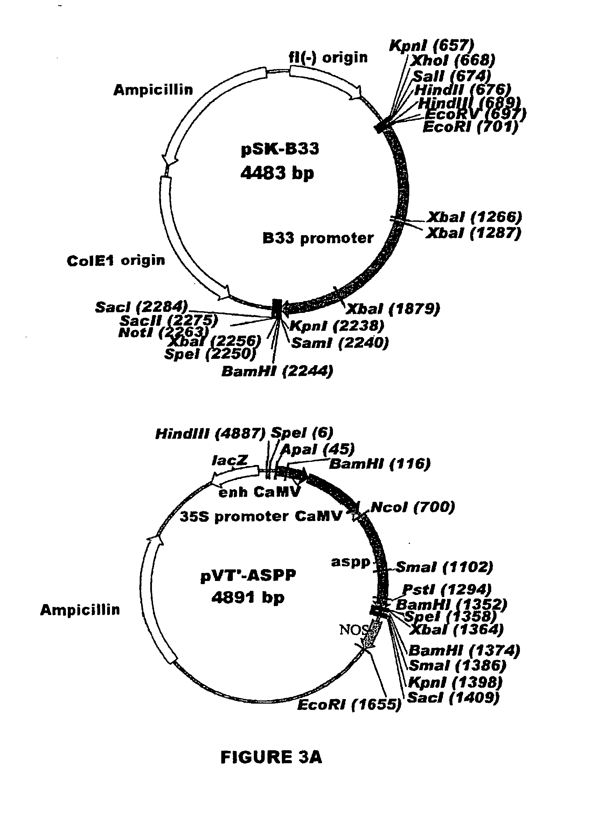

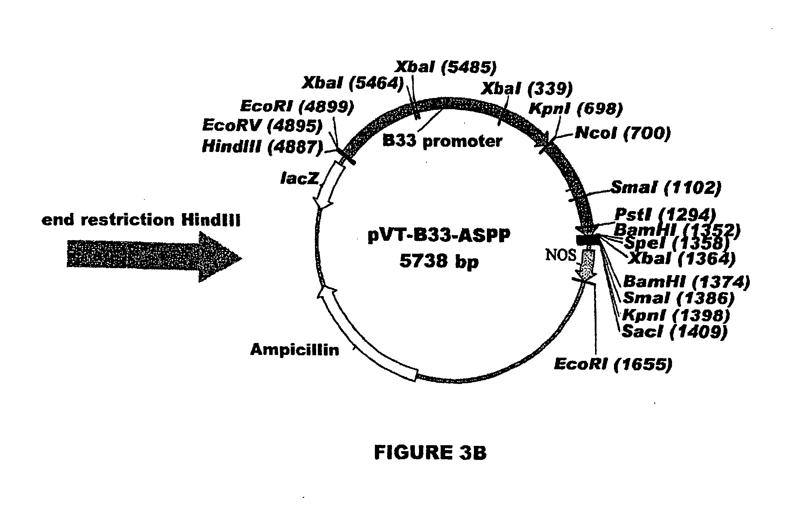

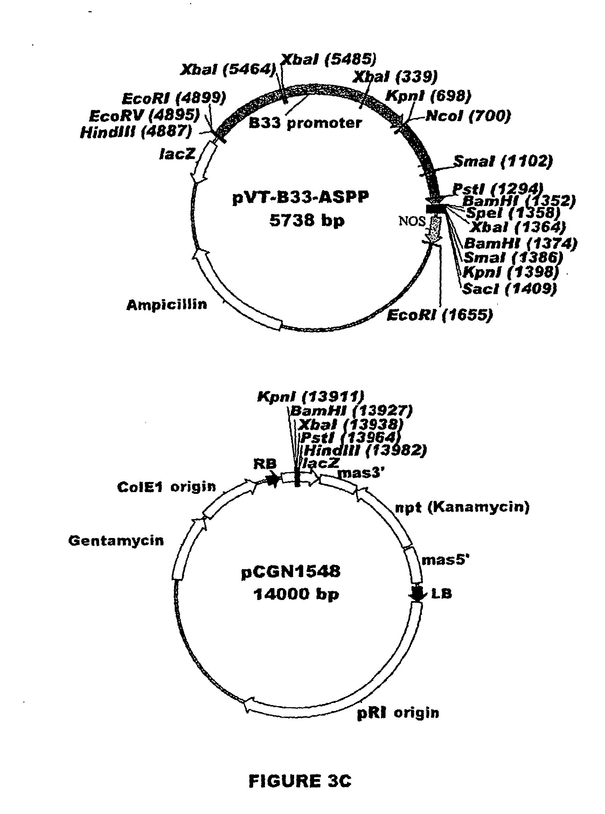

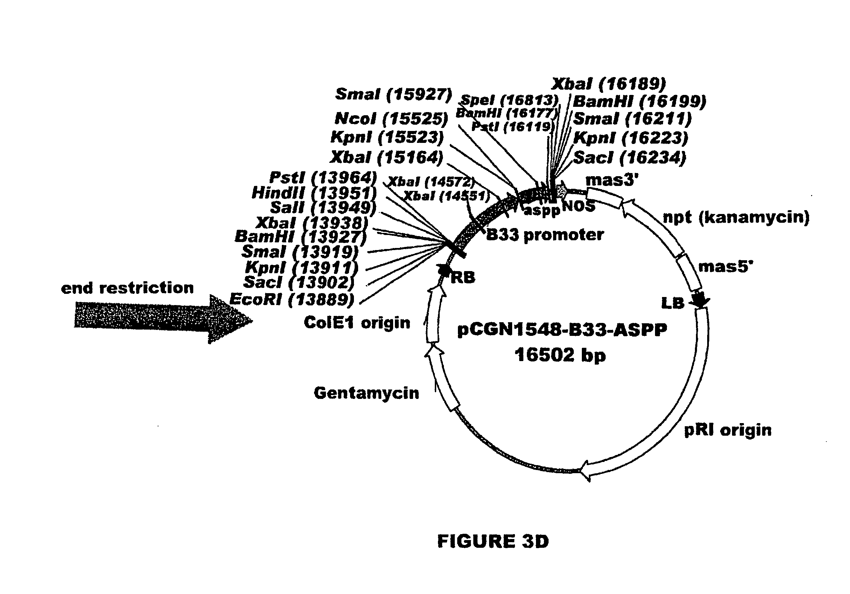

The plasmid pSK-B33 that contains the promoter of the gene that codes for the patatin gene (Rocha-Sosa, M., Sonnewald, U., Frommer, W., Stratmann, M., Schell, J., Willmitzer, L. (1989) "Both developmental and metabolic signals activate the promoter of a class I patatin gene" EMBO J. 8, 23-29) was digested with the enzymes HindIII and SmaI. The fragment released was cloned in pVT'-ASPP (Fig. 3A) after it had been digested successively with NcoI, T4 DNA polymerase and HindIII, giving rise to the plasmid pVT-B33-ASPP (Fig. 3B). For cloning this construction in a binary plasmid, pVT-B33-ASPP was digested successively with EcoRI and T4-DNA polymerase. The fragment released was cloned in pCGN1548 (Fig. 3C) after it had been digested successively with HindIII and T4 DNA polymerase. The plasmid thus obtained was given the name pCGN1548-B33-ASPP (Fig. 3D).

### c. Plants that overexpress aspP constitutively and accumulate AGPPase in the plastid

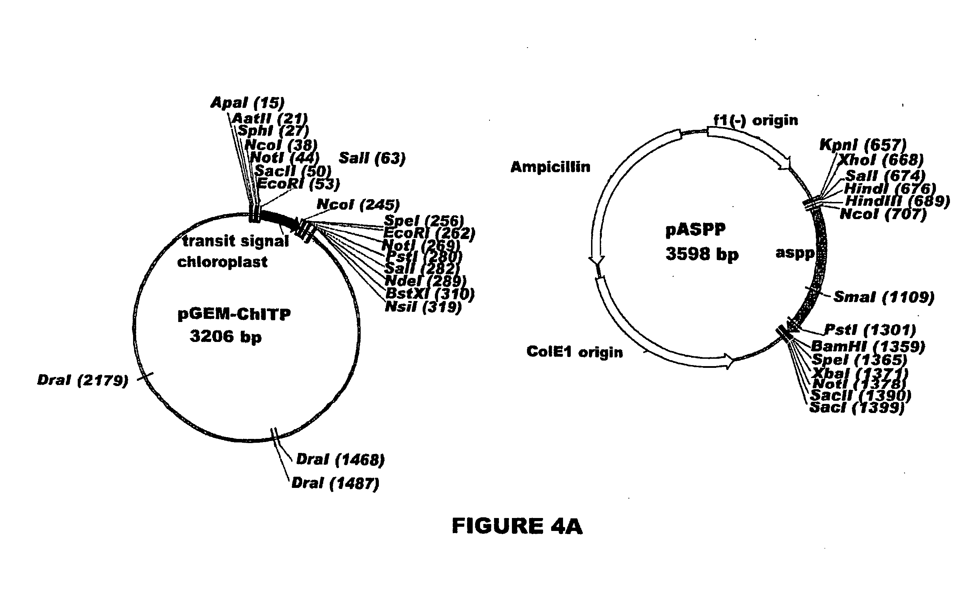

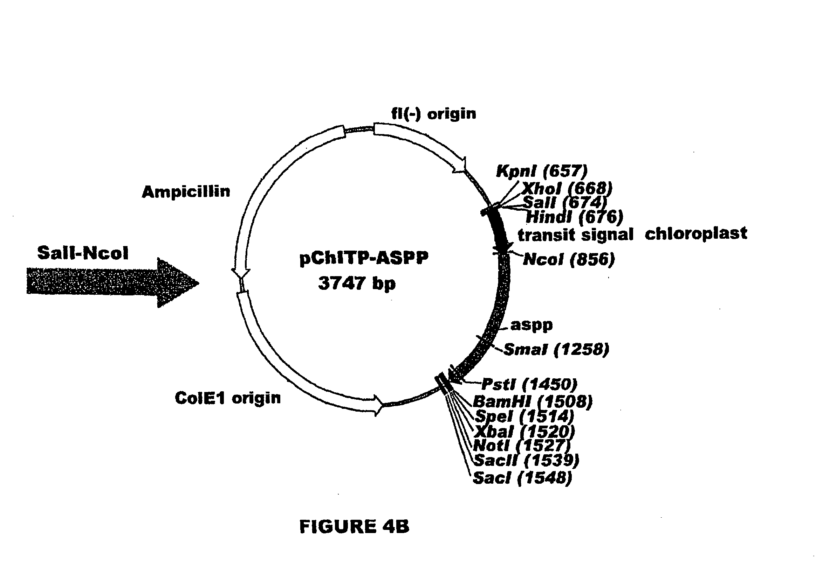

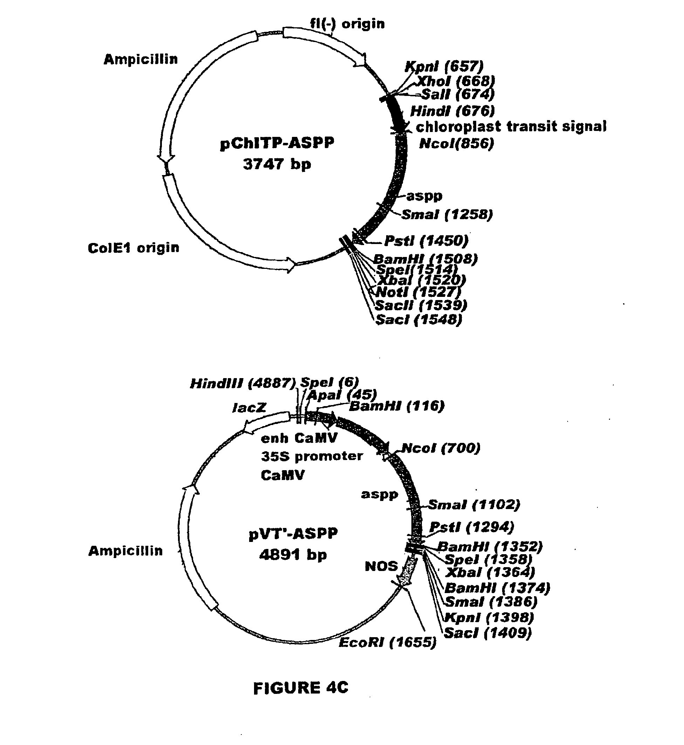

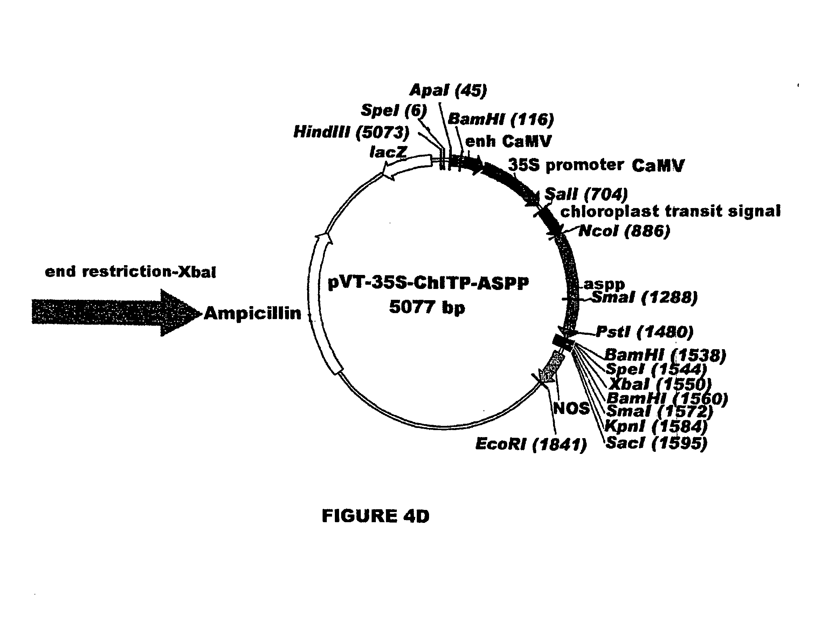

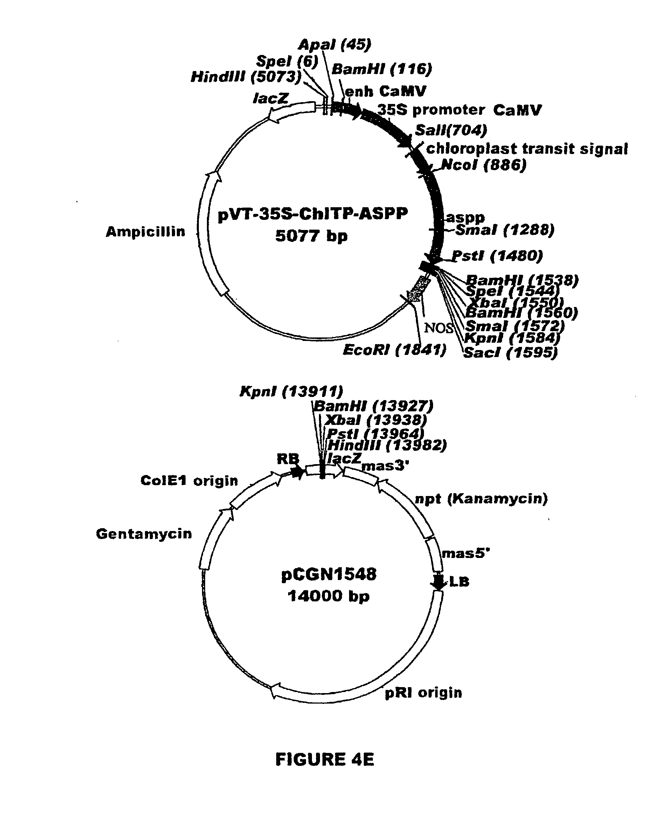

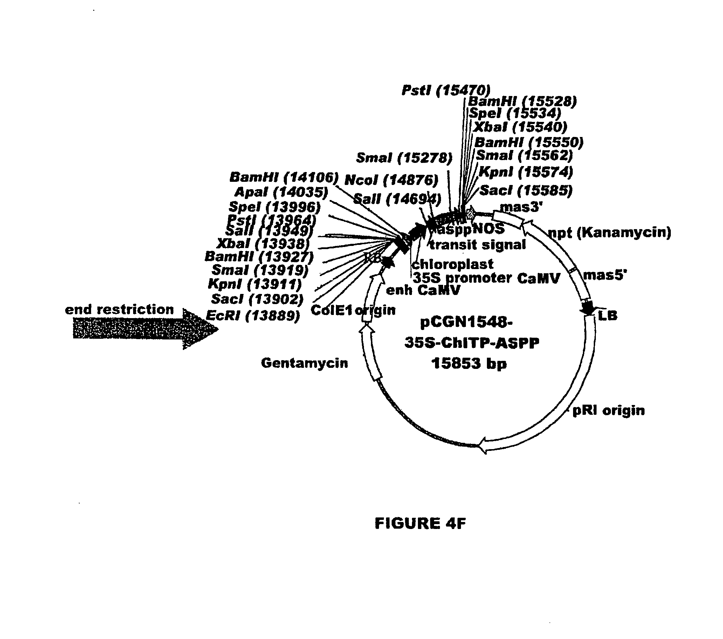

The plasmid pGEM-ChlTP (Fig. 4A) containing the region of the gene that codes for the transit peptide for transport of the P541 protein to the chloroplast (Houlné, G., Schantz, M-L., Meyer, B., Pozueta-Romero, J., Schantz, R. (1994) "A chromoplast-specific protein in Capsicum annuum: characterization and expression of the corresponding gene" Curr. Genet. 26, 524-527) was digested with SalI and NcoI. The fragment released was cloned in pASPP after it had been digested with SalI and NcoI, giving rise to the plasmid pChlTP-ASPP (Fig. 4B). pChlTP-ASPP was digested successively with Sail, T4 DNA polymerase and XbaI. The fragment released was cloned in pVT'-ASPP (Fig. 4C) that had previously been digested successively with NcoI, T4 DNA polymerase and XbaI, giving rise to the plasmid pVT-35S-ChlTP-ASPP (Fig. 4D). For cloning this construction in a binary plasmid, pVT-35S-ChlTP-ASPP was digested successively with HindIII, EcoRI and T4-DNA polymerase. The fragment released was cloned in pCGN1548 (Fig. 4E) after it had been digested successively with HindIII and T4 DNA polymerase. The plasmid thus obtained was given the name pCGN1548-35S-ChlTP-ASPP (Fig. 4F).

### Determination of the content of soluble sugars and of starch

The soluble sugars were extracted using the techniques described in the scientific literature (Heim, U., Weber, H., Baumlein, H., Wobus, U. (1993) "A sucrose-synthase gene of V. Faba L. Expression pattern in developing seeds in relation to starch synthesis and metabolic regulation" Planta 191, 394-401). Sucrose, fructose and glucose were determined using a DIONEX automated ion chromatograph connected to a CarboPac PA10 column, an ED50 electrochemical detector, a GP50 E1 gradient pump and an E01 eluent organizer. ADPG was determined using a HPLC system connected to a Partisil-10-SAX column (Rodríguez-López, M., Baroja-Fernández, E., Zandueta-Criado, A., Pozueta-Romero, J. (2000) "Adenosine diphosphate glucose pyrophosphatase: a plastidial phosphodiesterase that prevents starch biosynthesis". Proc. Natl. Acad. Sci., 97, 8705-8710). Starch was measured using commercial kits described in the literature (Rodríguez-López, M., Baroja-Fernández, E., Zandueta-Criado, A., Pozueta-Romero, J. (2000) "Adenosine diphosphate glucose pyrophosphatase: a plastidial phosphodiesterase that prevents starch biosynthesis". Proc. Natl. Acad. Sci. USA 97, 8705-8710) which are based essentially on the action of glucose oxidase on the glucose units of the starch followed by spectrophotometric detection of the NADH produced after a coupled enzymatic reaction.

### Development of an assaying device (kit) for determination of ADPG in extracts of plant or bacterial origin

The kit designed for the determination of ADPG is based on the action of the product with AGPPase activity on the ADPG molecule, releasing equimolar quantities of AMP and glucose-1-phosphate (G1P) that are easily detected by spectrophotometric methods. G1P is submitted to the action of the enzyme phosphoglucomutase, yielding glucose-6-phosphate (G6P), which in its turn can be made to react in a coupled manner with NAD+ by the action of the enzyme G6P dehydrogenase, obtaining 6-phosphogluconate and NADH, which can easily be determined by spectrophotometry at 340 nm.

### Production of specific polyclonal antibodies of the AGPPase of E. coli

Two milligrams of purified AGPPase was separated electrophoretically in SDS-PAGE. After it had been eluted, it was mixed with complete Freund's adjuvant (at 50%/50% ratio) and was then divided into three equal aliquots, each of which was injected in a rabbit for periods of two weeks. Approximately two months after the first injection, the rabbit's blood serum was extracted, which contains the specific polyclonal antibodies of AGPPase.

### Identification of the product using Western blotting

Samples of proteins from wild plants and transgenic plants that overexpress the gene aspP were separated in 10% SDS-PAGE. Then they were transferred to nitrocellulose membranes and the AGPPase was detected using the specific anti-AGPPase antibody according to the methodology described in the literature (Towbin, H., Staehelin, T., Gordon, J. (1979) "Electrophoretic transfer of proteins from polyacrylamide gels to nitrocellulose sheets: procedures and some applications". Proc. Natl. Acad. Sci. USA 76, 4350-4354).

### EXAMPLES OF APPLICATION OF THE INVENTION

Examples are described below in which the method of production and purification of the AGPPase is shown in detail. Other examples illustrate the use of AGPPase for the production of kits (assay devices) for determining ADPG in plant tissues. Another example shows the production of aspP. Finally, other examples describe the production of transgenic plants and bacteria that overexpress aspP.

### Example 1: Extraction and purification of AGPPase

All the steps were carried out at 4°C, unless indicated otherwise. Thirty liters of E. coli BL21 were centrifuged at 10,000 g and the bacterial pellet was resuspended in 200 ml of Tris-HCl 50 mM, pH 7.5. After sonication, the raw bacterial extract was centrifuged at 100,000 g for 30 minutes and the supernatant was heated at 58°C for ten minutes. After centrifugation at 30,000 g, the AGPPase present in the supernatant was precipitated with 30-50% of ammonium sulfate. The precipitate obtained after 30 minutes of centrifugation at 30,000 g (20°C) was resuspended in 2.7 ml of Tris-HCl 50 mM, pH 7.5. The sample was then submitted to gel filtration in a column of Superdex 200 (Pharmacia LKB Biotechnology, Uppsala, Sweden) pre-equilibrated with Tris-HCl 50 mM pH 7.5 and NaCl 150 mM. Elution was carried out with the same buffer. The fractions with AGPPase activity were combined, concentrated and applied to a type mono-Q ion-exchange column (XR 5X5, Pharmacia). After gradient elution with KCl, the active fractions were combined, dialyzed, concentrated and submitted to isoelectric focusing using a Rotofor Cell (Bio-Rad). The active fractions had a pI of approx. 5.2, and were combined, concentrated and used for electrophoretic separation in SDS-PAGE.

### Example 2: Enzyme assays

Unless indicated otherwise, all the enzyme reactions took place at 37°C. The determinations of AGPPase activity were performed using spectrophotometric determination of G1P in two steps as described by Sowokinos (1981) (Sowokinos, 1981, Plant Physiol. 68, 924-929). The reaction mixture contained Tris-HCl 50 mM pH 7.5, 5 mM MgCl2, the specified quantity of ADPG and the protein extract in a total volume of 50 microliters. All the assays were performed relative to a target of ADPG. After 20 minutes of incubation, the reaction was stopped by boiling in a dry bath for 2 minutes. The mixture was centrifuged at 20,000 g for 5 minutes and the supernatant was recovered. In the next step, G1P was determined spectrophotometrically in 300 microliters of mixture containing Hepes 50 mM pH 7, EDTA 1 mM, MgCl2 2 mM, KCl 15 mM, NAD+ 0.6 mM, one unit of phosphoglucomutase and another of G6P dehydrogenase from Leuconostoc mesenteroides, and 30 microliters of the supernatant resulting from step one. After 20 minutes of incubation, production of NADH was monitored at 340 nm using a Multiskan EX spectrophotometer (Labsystems). The amount of NADH produced by any protein extract in the absence of ADPG in step one was negligible.

The native molecular weight of the AGPPase was determined by gel filtration, by plotting the partition coefficient (Kav) against the logarithm of the molecular weight of the following reference proteins: bovine thyroglobulin (670 kDa), bovine gamma-globulin (158 kDa), ovoalbumin (45 kDa), myoglobin (17 kDa) and vitamin B-12 (1.3 kDa). The protein content was determined by Bradford's method using the reagent made by Bio-Rad and gamma-globulin as standard.

Table 1 below shows the purification of bacterial AGPPase. The unit (U) is defined as the quantity of enzyme that catalyzes the production of 1 µmol of product per minute.

**Table 1**

| | Total volume (mL) | Total protein (mg) | Total activity (mU) | Specific activity (mU/mg protein ) | Purific ation (factor) | Yield (%) |
|---|---|---|---|---|---|---|
| Raw extract | 198 | 4206 | 12 905 | 3 | - | 100 |
| Supernatant 100,000 g 58°C | 166 | 462 | 54 499 | 118 | 38 | 422 |
| Ammoniu m sulfate 50% | 2.7 | 101 | 29 719 | 293 | 95 | 155 |
| Superde x 200 | 15 | 7.4 | 19 125 | 2584 | 842 | 148 |
| Mono-Q | 2.7 | 1.9 | 6023 | 3170 | 1032 | 47 |
| Isoelec tric focusin g | 0.6 | 0.2 | 1807 | 9511 | 3098 | 15 |

### Example 3: Identification of the product with enzymatic activity obtained

The product with AGPPase activity thus obtained meets the following characteristics:
- The bacterial AGPPase is a pyrophosphatase that catalyzes the hydrolysis of ADPG producing equimolar quantities of G1P and AMP.
- In addition to ADPG, it also hydrolyzes ADPribose and ADPmannose.
- It does not hydrolyze molecules with phosphomonoester bonds such as G1P, G6P, AMP, 3-phosphoglycerate, and the like. Nor does it hydrolyze cyclic AMP or long-chain nucleic acids such as DNA and RNA.
- It requires magnesium for optimum functioning.
- It does not hydrolyze bis-para-nitrophenyl-phosphate, which is a substance that can be hydrolyzed by phosphodiesterases.
- Inhibited by phosphorylated molecules such as AMP, ADP, ATP, 3-phosphoglycerate, inorganic pyrophosphate, and others with similar characteristics, but not by orthophosphate.
- Inhibited strongly by molybdate.
- Its activity is adversely affected by the action of β-mercaptoethanol, EDTA, reduced cysteine, reduced glutathione, glutamate, aspartate, ascorbate, and other reducing and chelating agents.
- Michaelis-Menten constant (Km) for ADPG: 0.15 mMolar.
- Apparent molecular weight of the protein purified in denaturing gels, around 26 kDa.
- Apparent molecular weight measured by gel filtration, around 40-50 kDa, indicating that it may be a homodimer.
- In the present invention, characterization of the amino acid sequence provides us with another series of characteristics, such as:

- The amino acid sequence obtained by the Edman degradation is:
   - N-terminal end: SEQ ID NO: 1

### Example 4: Obtaining the gene that codes for AGPPase

Knowledge of the nucleotide sequence of the gene that codes for AGPPase of E. coli, aspP, made it possible to create two specific primers whose sequences are, in the 5' - 3' direction, SEQ ID NO: 2 and SEQ ID NO: 3. Using these primers, a DNA fragment was amplified by conventional PCR methods from the chromosomal DNA of E. coli BL21, which was inserted in a plasmid pSK Bluescript (Stratagene) which was amplified in the host bacterium XL1 Blue. The nucleotide sequence of the DNA is SEQ ID NO: 4 and the derived amino acid sequence is SEQ ID NO: 5.

### Example 5: Obtaining AGPPase from E. coli BL21 (DE3) that overexpresses aspP

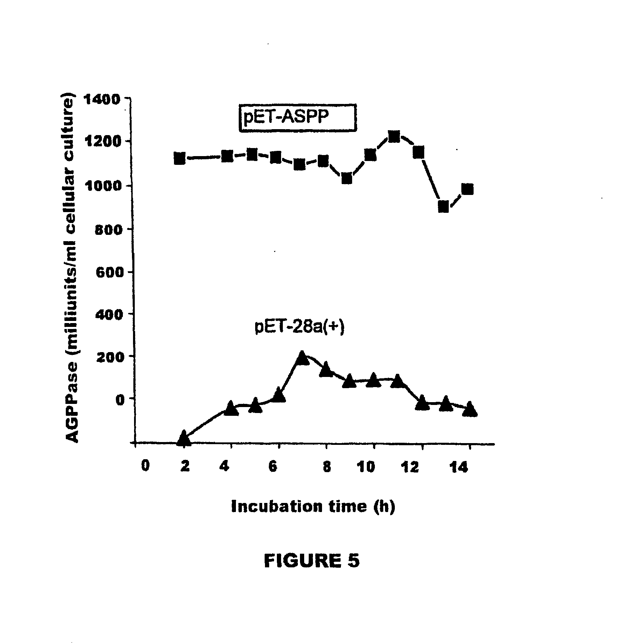

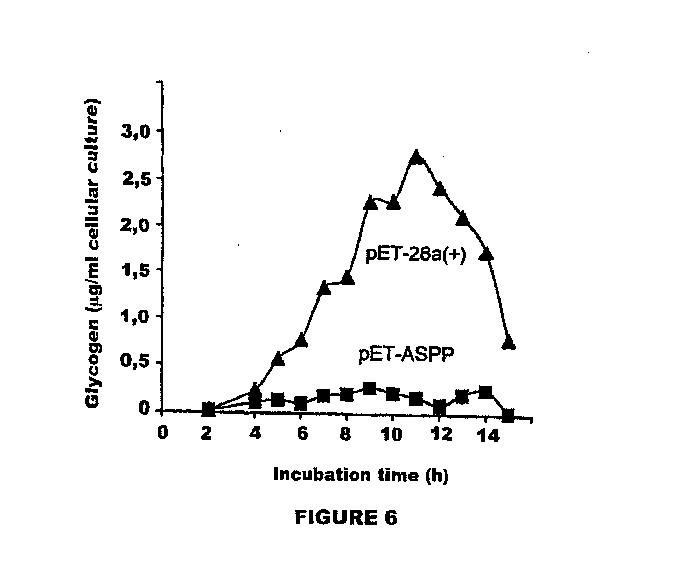

The induction of aspP in BL21 (DE3) bacteria transformed with pET-ASPP took place on adding 1 mM IPTG. After six more hours of culture, it was observed that the bacteria transformed with pET-ASPP had a significantly higher AGPPase activity than the controls transformed with the plasmid pET-28a (+) (see Fig. 5). The bacteria transformed with pET-ASPP were collected, resuspended in the buffer specified by the "His-bind" protein purification kit (Novagen), and were lysed by sonication. The supernatant obtained after centrifugation of the lysate was passed through an affinity column of Novagen's "His-bind" protein purification kit. The protein obtained had a size of 30 kDa in denaturing gels, which is slightly higher than the 26 kDa of the protein produced by the bacterium itself, owing to addition of a histidine glue. The purified protein had kinetic properties and substrate specificity identical to those described for the AGPPase produced by untransformed bacteria. On the other hand, the glycogen levels of the bacteria transformed with pET-ASPP were practically undetectable and significantly lower than the control bacteria (Fig. 6) .

### Example 6: Production of transgenic plants of tobacco, potato and tomato that overexpress AGPPase

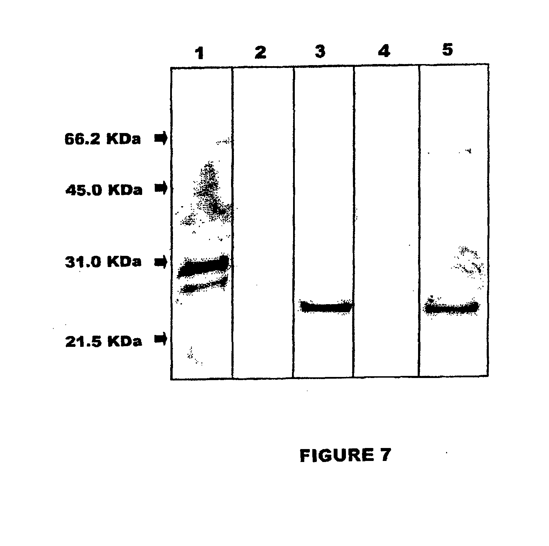

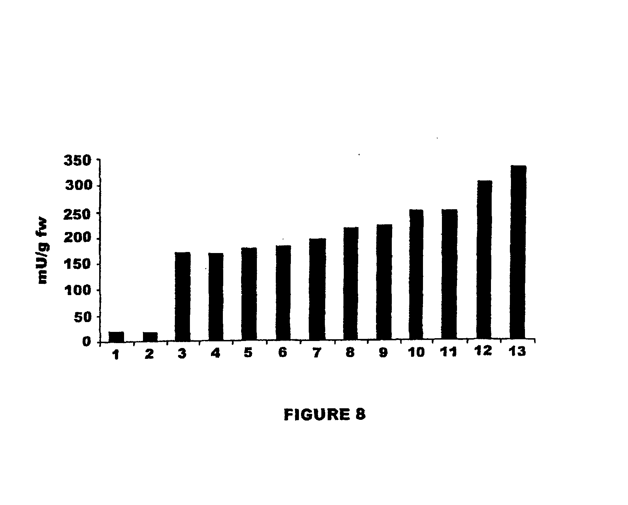

Using the strain of Agrobacterium tumefaciens CECT 5837, plants of tobacco (Nicotiana tabacum), potato (Solanum tuberosum) and tomato (Lycopersicon sculentum) that overexpress aspP were obtained. The protein extracts from these plants possess AGPPase of E. coli, as is demonstrated by detection of the Western blot type using specific antibodies of the AGPPase of E. coli (Fig. 7). The plants that overexpress aspP are characterized in that they possess AGPPase activity in all the organs analyzed (root, leaf, fruits, tubers and stem) of the order of 20-30 times greater than the endogenous AGPPase present in the untransformed plants (Fig. 8) . The plants that overexpress aspP had the following characteristics:
1. Low starch content.
2. High content of soluble sugars such as sucrose, glucose and fructose and low content of ADPG.
3. The external morphology of the plants that overexpress aspP is not aberrant, when compared with that of the untransformed plants.
4. The plants that overexpress aspP displayed great resistance to high temperatures and high concentrations of salts in the culture medium.

### Example 7: Development of enzyme kits for determination of ADPG

A kit containing the following elements has been developed for the determination of ADPG:
a. AGPPase obtained from BL21 (DE3) bacteria transformed with pET-ASPP, and purified using the "His-Bind" kit (Novagen).
b. NAD
c. Phosphoglucomutase (PGM)
d. G6P dehydrogenase (G6PDH)
e. Buffer

Determination of the amount of ADPG present in the test sample is based on spectrophotometric determination of the NADH produced according to the following coupled reaction:

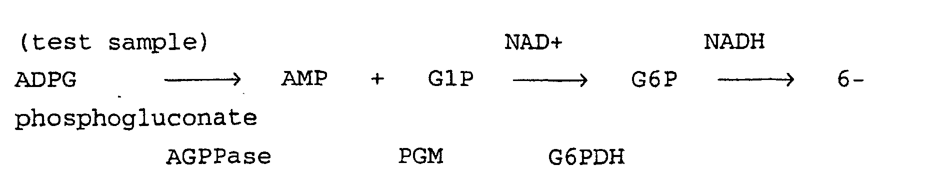

The amount of ADPG in a test sample would be determined by preparing a cocktail with the following composition (for 1 ml):
- Test sample
- 1 U of AGPPase
- 1 U of PGM
- 1 U of G6PDH
- 0.6 mM NAD
- Buffer Tris-HCl 50 mM pH 7.5, MgCl2 5 mM
- Water (to give 1 ml)

It is incubated at 37°C for 20 minutes, and the change in absorbance of the sample at 340 nm is observed. A cocktail that does not contain AGPPase can be used as a negative control.

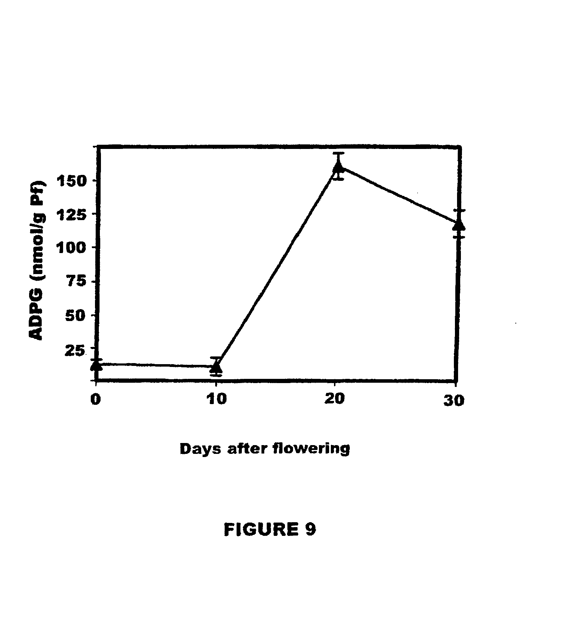

Fig. 9 illustrates the application of the kit for determining the ADPG contents at different stages of development of the endosperm of barley, which proved to be significantly identical to those determined using the chromatographic techniques described in the introduction.

### DESCRIPTION OF THE FIGURES

Fig. 1. Stages in construction of the plasmid pET-ASPP
Fig. 2. Stages in construction of the plasmid pCGN154835SAPP
Fig. 3. Stages in construction of the plasmid pCGN1548-B33-ASPP
Fig. 4. Stages in construction of the plasmid pCGN1548-35S-ChlTP-ASPP
Fig. 5. AGPPase activity
   Abscissa: incubation time, hours
   Ordinate: milliunits/ml of cellular culture.
Fig. 6. Glycogen levels in the control bacteria (pET-28a(+) ) relative to the transformed bacteria pET-ASPP.
   Abscissa: incubation time, hours
   Ordinate: µg of glycogen/ml cellular culture
Fig. 7. Immunodetection of AGPPase in protein extracts from leaves and tubers of wild potato plants (lines 2 and 4) and potato plants that overexpress aspP (lines 3 and 5). Line 1: AGPPase purified after being overexpressed in E. coli; Lines 2 and 3: extracts from leaves; Lines 4 and 5: extracts from tubers.
Fig. 8. Hydrolytic activity of ADPG in raw extracts from tubers of wild potato plants (columns 1 and 2) and in various clones of plants that overexpress aspP (columns 3 to 13).
Fig. 9. Determination of ADPG content at various stages of development of barley endosperms.
   Abscissa: Days after flowering
   Ordinate: ADPG content

## Claims

1. A method of production of an enzyme product of microbial origin with ADPG pyrophosphatase (AGPPase) activity, **characterized in that** a suspension of E. coli is submitted to extraction of the protein fraction by a buffer, sonication of the extract, followed by a purification procedure by successive centrifugations and precipitations, with adjustments both of the pH and of the ionic strength of the medium, preferably including heating of the protein at 58°C and cooling in ice, and purification by gel filtration, isoelectric focusing, ion exchange or other equivalent methods of purification of proteins extracted from bacteria.

2. The method as claimed in claim 1 that comprises the following steps: (1) Culture of E. coli BL21 in 30 liters of M9 minimum medium with 5 mMolar of glucose, (2) sedimentation of the bacteria by means of centrifugation, (3) resuspension in Tris-HCl 50 mM, pH 7.5 and MgCl2 5 mM, (4) sonication, (5) ultracentrifugation at 100,000 g, (6) heating of the supernatant for 10 minutes at a temperature of 58°C, followed by cooling in ice, (7) centrifugation and concentration of the protein of the supernatant by precipitation in ammonium sulfate and resuspension in Tris-HCl 50 mM pH 7.5 and (8) purification by gel filtration chromatography, ion exchange or isoelectric focusing.

3. The method as claimed in claim 1, **characterized in that** the strain of E. coli used expresses a DNA fragment, represented by SEQ ID NO: 4, amplified by PCR from the genomic DNA of E. coli BL21 by means of two primers obtained in their turn from the 5' and 3' regions, represented by SEQ ID NO: 2 and SEQ ID NO: 3 respectively, of the gene that codes for AGPPase.

4. The method as claimed in any one of claims 1 and 3, **characterized in that** the AGPPase is obtained and purified from strains of CECT5357.

5. An enzyme product of prokaryotic microbial origin, designated AGPPase, obtainable according to the method of any one of claims 1 to 4.

6. An enzyme product of prokaryotic microbial origin, designated AGPPase, **characterized in that** it displays ADPG pyrophosphatase (EC 3.6.1.21) enzymatic activity that catalyzes the hydrolysis of ADPG in equimolar fashion to G1P and AMP, does not hydrolyze molecules with phosphomonoester bonds nor mono-, di- or triphosphate nucleotides such as, among others, ATP, UTP, ADP, UMP or AMP, requires MgCl2, is not capable of hydrolyzing bis-p-nitrophenyl-phosphate, is inhibited by molybdate and phosphorylated molecules, its activity is adversely affected by reducing and chelating agents, it is very stable at pH between 7.5 and 8.5, and, in addition to ADPG, recognizes ADPribose and ADPmannose but not other nucleotide sugars such as UDPglucose or GDPmannose, among others.

7. The enzyme product as claimed in claims 5 and 6, **characterized in that** it does not hydrolyze, among others, G1P, G6P, AMP, 3-phosphoglycerate, CAMP, nor long-chain nucleic acids, such as DNA or RNA.

8. The enzyme product as claimed in claims 5 to 7, **characterized in that** it is inhibited by inorganic pyrophosphate, and phosphate esters such as, among others, AMP, ADP, ATP, or 3-phosphoglycerate.

9. The enzyme product as claimed in claims 5 to 8, **characterized in that** its activity is adversely affected by, among others, β-mercaptoethanol, EDTA, reduced cysteine or ascorbate.

10. The enzyme product as claimed in claims 5 to 9, **characterized in that** it does not recognize as substrates, among others, UDPglucose, GDPglucose, adenosine 5'-phosphosulfate, or bis-p-nitrophenyl-phosphate.

11. The enzyme product as claimed in claims 5 to 10, **characterized in that** it has an apparent molecular weight determined by gel filtration of about 40-50 kDa, as well as exhibiting a Km for ADPG of 0.15 mMolar.

12. The enzyme product as claimed in claims 5 to 11, **characterized in that** it contains in its sequence at least one polypeptide fragment shown in SEQ ID NO: 1.

13. The enzyme product as claimed in claims 5 to 11, **characterized in that** it consists of SEQ ID NO: 5 or homologous sequences resulting from the degeneration of the genetic code.

14. Use of the enzyme product of claims 5 to 13 in the manufacture of assay devices and/or compositions for application in the determination of ADPG.

15. An assay device for the determination of ADPG, **characterized in that** it includes the enzyme product of claims 5 to 13, in such a way that determination is based on the G1P released during the reaction catalyzed by AGPPase.

16. The assay device as claimed in claim 15, **characterized in that** determination is based on the G1P released, which is submitted to the enzyme phosphoglucomutase to produce G6P, which is in its turn reacted in coupled fashion with NAD+, by the action of the enzyme G6P dehydrogenase, obtaining 6-phosphogluconate and NADH, determinable by conventional methods: spectrophotometric, fluorimetric or of some other nature.

17. DNA sequence selected from:
a) a sequence that codes for a polypeptide that includes the amino acid sequences represented by SEQ ID NO: 1 or SEQ ID NO: 5;
b) a sequence that includes the polynucleotide sequence represented by SEQ ID NO: 4;
c) complementary sequences that hybridize with the sequences defined in a) or b) and that code for an enzyme product with AGPPase activity;
d) sequences that differ from those defined in c) owing to degeneration of the genetic code.

18. Use of the DNA sequences of claim 17 in the production of transgenic plants that overexpress the enzyme AGPPase.

19. The method of production of transgenic plants that overexpress AGPPase, **characterized in that** it uses a transformation vector that contains a plasmid that includes some of the DNA sequences of claim 17.

20. The method of production of transgenic plants as claimed in claim 19, **characterized in that** the transformation vector is Agrobacterium tumefaciens.

21. The method of production of transgenic plants as claimed in claim 20, **characterized in that** the transformation vector comprises strains of Agrobacterium tumefaciens CECT5901.

22. Transgenic plants with reduced starch content and resistant to high salinity and temperatures, obtainable according to the method of claims 19 to 21, **characterized in that** they overexpress the enzyme AGPPase.

23. Use of the DNA sequences of claim 17 in the production of transformed bacteria that overexpress AGPPase.

24. A method of obtaining transformed bacteria that overexpress AGPPase, **characterized in that** it uses a transformation vector, in particular a plasmid that includes some of the DNA sequences of Claim 17.

25. Transformed bacteria with reduced glycogen content obtainable according to the method of claim 24, **characterized in that** they overexpress the enzyme AGPPase.

26. Use of the transformed bacteria of claim 25 in the feeding of animals, especially in the feeding of fish.


LIST OF SEQUENCES

<110> PUBLIC UNIVERSITY OF NAVARRA
<120> "Bacterial ADPglucose pyrophosphatase, method of
production, use in the manufacture of testing devices and
in the production of transgenic plants and bacteria".
<130> PCT-104
<160> 9
<210> 1
<211> 20
<212> Peptide
<213> Escherichia coli BL21
<220>
<223> N-terminal end of AGPPase

<400>
Met Val Lys Pro Asp Ser Leu Pro Val Thr Phe Gly Lys Asn Asp
                5                   10                  15
Val Glu Ile Ile Ala
                20

<210> 2
<211> 30
<212> DNA
<213> Escherichia coli BL21
<220>
<223> Primer of the 5' region of aspP

<400>
atggttaagccagacagcctgcccgttaca       30

<210> 3
<211> 30
<212> DNA
<213> Escherichia coli BL21
<220>
<223> Primer of the 3' region of aspP

<400>
ttatgcccactcatttttaacgcttgatg 30

<210> 4
<211> 233
<212> DNA
<213> Escherichia coli BL21
<220>
<223> aspP

<400>
atg gtt aag cca gac agc ctg ccc gtt aca ttt ggc aaa aac 42
gat gta gaa att att gca cga gaa aca ctt tat cgc ggc ttt 84
ttt tca tta gat ctt tat aga ttt cgt cat cgt cta ttc aac 126
ggg caa atg agt cat gag gta cgg cgg gaa att ttt gag cgc 168
ggt cac gcc gca gtc ttg cta ccc ttt gac cca gtg cgt gat 210
gaa gtt gtg ctg att gag cag att cgg att gcc gcg tac gac 252
acc agc gaa acc ccc tgg cta ctg gag atg gtt gcc ggg atg 294
att gaa gag ggt gaa agt gtg gaa gat gtt gcc cgt cgc gaa 336
gcg att gaa gag gcg gga ctg ata gtc aaa cgg acc aaa ccg 378
gtg tta agt ttc ctg gca agc ccg ggg ggc acc agt gag cgt 420
tcg tca att atg gtg ggc gaa gtg gac gcc acg acc gca agc 462
ggt att cat ggt ctg gct gat gaa aac gaa gat att cgc gtt 504
cat gtg gta agc cgg gaa cag gca tac cag tgg gta gaa gag 546
ggg aaa atc gac aac gca gcg tcg gtc atc gct ttg caa tgg 588
ctg cag ctg cat cat caa gcg tta aaa aat gag tgg gca taa 630

<210> 5
<212> protein
<213> Escherichia coli BL21
<220>
<223> AGPPase derived from aspP
<400>

Met Val Lys Pro Asp Ser Leu Pro Val Thr Phe Gly Lys Asn Asp Val Glu Ile Ile Ala
                5                   10                  15                  20
Arg Glu Thr Leu Tyr Arg Gly Phe Phe Ser Leu Asp Leu Tyr Arg Phe Arg His Arg Leu
                25                  30                  35                  40
Phe Asn Gly Gln Met Ser His Glu Val Arg Arg Glu Ile Phe Glu Arg Gly His Ala Ala
                45                  50                  55                  60
Val Leu Leu Pro Phe Asp Pro Val Arg Asp Glu Val Val Leu Ile Glu Gln Ile Arg Ile
                65                  70                  75                  80

Ala Ala Tyr Asp·Thr Ser Glu Thr Pro Trp Leu Leu Glu Met Val Ala Gly Met Ile Glu
85 90 95 100
Glu Gly Glu Ser Val Glu Asp Val Ala Arg Arg Glu Ala Ile Glu Glu Ala·Gly Leu Ile
105 110 115 120
Val Lys Arg Thr Lys Pro Val Leu Ser Phe Leu Ala Ser Pro·Gly Gly Thr Ser Glu Arg
125 130 135 140
Ser Ser Ile Met Val Gly Glu Val Asp Ala Thr Thr Ala Ser Gly Ile His Gly Leu Ala
145 150 155 160
Asp Glu Asn Glu Asp Ile Arg·Val His Val Val Ser Arg Glu Gln Ala Tyr Gln Trp Val
165 170 175 180
Glu Glu Gly Lys·Ile Asp Asn Ala Ala Ser Val Ile Ala Leu Gln Trp Leu Gln Leu His
185 190 195 200
His Gln Ala Leu Lys Asn Glu Trp Ala ***
205 210